# EUROPEAN PATENT SPECIFICATION

(11) **EP 3 235 511 B1**
(45) Date of publication and mention of the grant of the patent: **19.12.2018**
(21) Application number: 17166846.0
(22) Date of filing: 18.04.2017
(51) Int. Cl.: A61K 45/06, A61K 31/10, A61K 31/136, A61K 31/63, A61K 31/635, A61P 31/22, A61P 43/00

(54) **SULPHONAMIDE COMPOUNDS FOR USE IN THE TREATMENT OF HHV-8-ASSOCIATED DISEASES**
SULFONAMIDVERBINDUNGEN ZUR BEHANDLUNG VON HHV-8 VERWANDTEN ERKRANKUNGEN
COMPOSÉS SULPHONAMIDES DESTINÉS AU TRAITEMENT DES MALADIES ASSOCIÉES AVEC HHV-8

(30) Priority: 18.04.2016 IT UA20162680
(43) Date of publication of application: 25.10.2017
(73) Proprietor: UNIVERSITA' DEGLI STUDI DI CAGLIARI, 09124 Cagliari (IT)
(72) Inventor: POMPEI, Raffaello, 09124 Cagliari (IT)
(74) Representative: Camolese, Marco

(56) References cited:
- WO-A1-2010/138652
- WO-A1-2011/053848

## Description

### Technical field

The present invention relates to sulphonamide compounds for use in the antiviral treatment of *Herpes 8* (or KSHV) virus in the latent stage, and/or diseases caused by, or attributable to, said virus, wherein said diseases are selected from Kaposi's sarcoma, pleural effusion lymphoma (PEL) and Castleman's disease.

### Prior art

Human *Herpes 8* virus (hereinafter, HHV8 or KSHV) is a lymphotropic virus ranked among the *gamma*-herpesviruses. In particular, it is the causative agent of Kaposi's sarcoma (KS) (a malignant angiosarcoma typically affecting immune-suppressed people, patients suffering from AIDS and transplant patients), pleural effusion lymphoma (PEL) and a series of lymphoproliferative diseases such as Castleman's disease. Like all the herpesviruses, KSHV also has the ability to cause an acute infection at first contact (in the so-called *lytic* stage) and then to enter into a latent infection stage, which can also persist for the whole lifetime of the subject.

In other words, the KSHV virus has two typical stages of reproduction, a so-called *lithic* stage (or acute, associated with the production of a large number of infectious virions), and a so-called latent stage, in which the virus remains anchored to the cell nucleus in the form of *episome* and is replicated only when the cell divides.

It is known that the KSHV virus in the lytic stage is sensitive (and hence is treatable with success) to common anti-herpetic drugs, such as *acyclovir, foscarnet, interferon.* However, when it is in the latent stage, the same virus proved to be substantially insensitive to any treatment so far tested (both with the above-mentioned drugs and with other known anti-viral agents).

The KSHV virus in its latent stage has been correlated with various degenerative systemic diseases; among these the most common are, for example, *Lupus erytematosus* and Multiple myeloma. Currently, Kaposi's sarcoma is treated as a classic tumour with the use of conventional chemotherapeutic agents (for example, anthracycline-based) and alpha-interferon. This therapy is able to control the progress of the disease, but does not cure the KSHV infection when the virus is in the latent stage as an *episome* bonded to cellular DNA; on the other hand, as already indicated above, the traditional anti-herpes drugs are able to suppress the virus replication **only** in its lytic stage, but **not** in its latent stage.

During the latent infection stage, herpes viruses express only a limited number of genes and antigens, but these are able to carry out important functions and in some cases can induce alterations in the physiology and cellular biochemistry of great biological relevance. In fact, the latent infectious status of some herpesviruses has been correlated with changes in cell metabolism and physiology that can lead to the onset of cancer or of degenerative diseases of certain organs or apparatus in humans (see Table 1).

Just as an example, *Herpes simplex 1* (HSV 1) is under study for a possible relation with diseases of the nervous system and with Alzheimer's disease; *Herpes varicella-zoster* has been considered the cause of chronic neurites and osteo-articular diseases; it is established that the Epstein-Barr virus is the cause of malignant lymphomas and has been associated with multiple sclerosis; *Herpes 6* (HHV 6) has been associated with chronic pathologies of the thyroid (auto-immune thyroiditis); in turn, the KSHV (or HHV 8) virus has been recognized as a cause of malignant sarcomas (SK) and chronic lymphoproliferative diseases (e.g., lymphomas; lupus; multiple myeloma).

**Table 1: Herpesvirus and correlation with chronic degenerative diseases**

| | |
|---|---|
| HSV 1 | Alzheimer's Disease |
| *Varicella-zoster* virus | Neurites & Osteo-articular diseases |
| Epstein-Barr virus | Lymphomas and Multiple sclerosis |
| HHV 6 | Auto-immune thyroiditis |
| KHSV (HHV 8) | Lymphomas; L*upus*; Multiple myeloma |

### Mechanisms of latency of the KSHV (or HHV 8) virus

The KSHV virus is typically a lymphotropic virus and its natural reservoir is formed by type B lymphocytes. Lymph cell lines are currently available (BC3 and BCBL1), collected from patients with pleural lymphomas (PEL), which are permanently infected with KSHV and are therefore experimentally useful as a virus source. The virus can be recovered from these cells in infectious form by treatment with TPA (tetra-phorbol-myristate acetate) that induces the passage of the KSHV virus from the latent stage to the one of lytic viral reproduction. The KSHV virus has no clear cytolytic activity and therefore, in order to highlight it in the cells, molecular or enzyme immunoassay methods are needed. In its latent stage, the KSHV virus can be highlighted using PCR (*Polymerase Chain Reaction*) amplification of the gene for the latency nuclear antigen (LANA), which is expressed by ORF73 gene. This factor allows the viral genome to remain anchored to the cellular genome (in a condition that is technically defined "*tethering*") in the form of episome, not integrated in the cell nucleus. During the latency stage, few viral antigens are expressed; among these, in addition to LANA, vCYCLIN (ORF71), FLIP (ORF74), and the various Kaposines (K12) are important. While vCYCLIN and FLIP are expressed in small quantities and have a predominantly nuclear function, Kaposines are produced in abundance and are also included in the cell membrane. The latent stage infection by KSHV (or HHV 8) causes deep changes in the biochemical and physiological functions of the cells. The most evident effects are, in particular: a change in cell permeability, resistance to stress and to lack of serum, resistance to toxic chemicals, increased glycolysis, increased insulin receptor expression and tumour transformations (e.g., Kaposi's sarcoma and neoplastic diseases of lymph cells).

### Herpes 8 virus inhibition systems

As known from literature, the KSHV virus has proved otherwise sensitive to a number of traditional anti-herpetic drugs, as exemplified in Table 2. These anti-herpetic drugs can inhibit the production of infectious virions in the lytic phase, but they do **not** interfere with the KSHV virus in its latency stage.

**Table 2: Action of traditional anti-herpetic drugs on KSHV virus**

| **Drug** | **Action on the *lytic* virus** | **Action on the *latent* virus** |
|---|---|---|
| Foscarnet | inhibition | no inhibition |
| Acyclovir | inhibition | no inhibition |
| Analogs of purine bases* | inhibition | no inhibition |
| Interferon *beta* | inhibition | slowing down and partial inhibition |
| Glycyrrhizic acid | inhibition | Apoptosis |

| | | |
|---|---|---|
| **: for example, cidofovir and ganciclovir* | | |

Some works have recently appeared that describe substances that would potentially affect the latency of herpes viruses. For example, Li N., et al., Discovery of Selective Inhibitors Against EBNA1 via High Throughput, In Silico Virtual Screening. PLoS ONE, 2010, 5(4), e10126, described some sulphonic derivatives which are able to prevent the attack of the EBNA1 latency factor of the Epstein-Barr virus to the cellular genome. In turn, Paul A.G., et al., Targeting KSHV/HHV-8 Latency with COX-2 Selective Inhibitor Nimesulide: A Potential Chemotherapeutic Modality for Primary Effusion Lymphoma. PLoS One, 2011, 6(9), e24379 described that nimesulide seems to possess a certain action in thwarting the attack of the LANA latency factor of the KSHV virus to the BC3 cells genome.

However, to the best knowledge of the Applicant, a fully satisfactory solution to the need to identify drugs adapted to specifically and in high percentage inhibit the KSHV virus in its latent form has not yet been identified, let alone described or suggested.

### Technical problem

It is therefore of great importance, and strongly felt in the medical profession, the need to have suitable drugs and the use thereof for the inhibitory treatment of the KSHV virus in its latent stage, thereby providing a possibility of treatment against diseases typical of this virus, in particular, tumours and those described above. The object of the present invention is to provide an adequate response to the technical problem highlighted above.

### Summary of the invention

The Applicant has now found that a number of known sulphonamide compounds used for their antibacterial activity are able to give an adequate response to the technical problem highlighted above.

Therefore, an object of the present invention are said compounds described above for use in the treatment of the KSHV virus in its latent stage, as shown in the appended independent claim.

Another object of the present invention are the pharmaceutical compositions of said derivatives described above for use in the treatment of the KSHV virus in its latent stage, as shown in the appended independent claim.

Another object of the present invention are said compounds and said pharmaceutical compositions described above for use in the treatment of the diseases caused by said KSHV virus, as shown in the appended claims.

Preferred embodiments of the present invention are set forth in the appended dependent claims.

The preferred embodiments of the present invention shown in the following description are described herein only by way of example.

### Brief Description of Drawings

**Figure 1** shows the results obtained by RT-PCR (Real Time - Polymerase Chain Reaction) which show that the sulphonamide compounds of the invention eliminate the DNA of the HHV 8 virus in its latent form from perpetually infected BC3 cells.
**Figure 2** shows a block diagram of the inhibitory activity of the sulphonamide compounds of the invention on the formation of the MDM2-p53 complex.

### Detailed Description of the Invention

It has now been unexpectedly found that a number of sulphonamide compounds have a potent specific inhibitory activity on the HHV 8 (KSHV) virus in its latent stage. These compounds have been characterized for the antiviral and cytotoxic activity *in vitro,* and their ability to interfere at the molecular level with the factors responsible for the latency state (LANA, p53, MDM2) has been studied and shown.

For example, said compounds have shown, on average, to be able to suppress the latency state in the HHV 8 (KSHV) virus in permanently infected lymphoma BC3 cells, after a brief period of contact. Other studies with these compounds revealed that they could interfere with the formation of the MDM2-p53 complex, which is necessary to the HHV 8 virus to remain linked to the cellular DNA in the episome state.

The present invention is therefore directed to a sulphonamide compound selected from the group consisting of sulphanilamide, sulphaguanidine, sulfathiazole, sulfamethoxazole and/or mixtures thereof, for use in the treatment of KSHV/HHV 8 virus in its latent stage and/or for use in a disease selected from Kaposi's sarcoma, pleural effusion lymphoma (PEL) and Castleman's disease.

In a particularly preferred embodiment, said sulphonamide compounds are to be taken individually as such or in combination with each other.

Another object of the present invention is a pharmaceutical composition comprising at least one of said sulphonamide compounds for use as described above.

Said pharmaceutical composition generally belongs to one of the ones that are substantially already known and commonly used in therapy at the concentrations approved by the health authorities for the uses already known (e.g. as antibacterials/antibiotics). Advantageously, it turned out that said compositions, already known, tested/studied and approved by the regulatory authorities, can also be applied to the treatment of the HHV 8 virus in the latent stage, preferably using the formulations already on the market, optionally making the minimum appropriate modifications that may be necessary.

In said above pharmaceutical composition, the at least one active ingredient is present in an effective amount in the range from 100 to 1,000 mg; preferably, from 200 to 800 mg; more preferably, from 400 to 800 mg.

As regards the dosage of the active ingredient(s), in general the dosages commonly administered in the antibacterial therapy with sulphonamides will be preferably used. In any case, by way of example, the daily dosage recommended for the sulphonamide active ingredient(s) of the present invention can range from 200 to 2,000 mg of active ingredient(s) *pro die* in 1-3 doses. Preferably, the daily dose can range from 400 to 1,200 mg *pro die* divided into 2 doses; more preferably, the daily dose can range from 400 to 800 mg *pro die* divided into 2 doses.

Likewise, the above pharmaceutical compositions will also contain the known amounts of excipients, carriers, fillers, additives and adjuvants as already used in the commercially available pharmaceutical compositions for antibacterial use.

The following experimental section is intended to demonstrate, by way of example, the ability of the sulphonamide compounds as described above of interfering with the HHV 8 (or KSHV) virus, by inhibiting it in the latent stage and eradicating the infection thereof in Kaposi's sarcoma, B-cell lymphomas, pleural effusion lymphoma (PEL) and in a series of lymphoproliferative diseases such as Castleman's disease.

### Experimental Section

### Materials and methods

### Cells and Viruses

A stock of human HUVEC umbilical cord primary cells (Invitrogen Life Technologies, UK) was cultured in a culture medium M200 (Gibco, Life Technologies, UK) with low serum growth supplement (LSGS, Invitrogen Life Technologies, UK). The HUVEC cells were always kept in a semi-confluent state and were sub-cultured at least once a week. Prior to testing, the cells were sub-cultured no more than 3-5 times. The BC3 cells permanently infected with HHV 8 were cultured in a RPMI-1640 medium supplemented with 10% fetal calf serum (FCS) (Invitrogen Life Technologies, UK). The BC3 cells were used to produce 100x concentrated stocks of HHV 8, as described in the literature. The pellet of the virus was suspended in RPMI, filtered through a 0.22 µm filter and stored at - 80 °C until use. The quantitative analysis of viral genomes present in the stock preparation was obtained by a real-time polymerase chain reaction (qPCR). Inocula without purified cells contained an average number of 4.7 x 10⁵ viral DNA copies/mL (data not shown). For infection of cells, approximately 5.0 x 10⁴ HUVEC cells/mL were seeded in 12 multi-well plates. The cells were infected with HHV 8, concentrated to a multiplicity of at least 10-20 genomes per cell in an M200 medium containing 2 µg/mL polibrene (esadimetrin bromide) for 2 hours at 37 °C. After 24-48 h, the infected cells were observed with an optical microscope to detect the typical spindle cell morphology. Only cell mono-layers with at least 70-80% of HHV 8 infected cells were used for the experiments.

The THP-1 cells were purchased from Life Technologies (UK) and were cultured in RPMI 1640 medium with 10% fetal bovine serum (FBS).

### Compounds

The sulphonamide compounds of the present invention used in this assay are, for example, commercial drugs purchased from Sigma-Aldrich Co. (St. Louis, USA). They were dissolved in DMSO to a concentration of 100 mg/mL and kept refrigerated until ready to use.

The product Nutlin 3, a cis-imidazoline analog which is known to inhibit the interaction between MDM2 and the p53 tumour suppressor, was purchased from Sigma-Aldrich and was used as a positive control.

### Cytotoxicity of the drugs

The cytotoxicity of the compounds under test was assessed by measuring the effect on the cell morphology and the cell viability *in vitro.* Cell mono-layers were prepared in 24-well tissue culture plates and exposed to various concentrations of the compounds under test. The plates were controlled by optical microscopy after 24, 48 and 72 h. Cytotoxicity was evaluated as morphologic changes (for example, rounding, contractions, detachments). The cell viability was determined by means of a tetrazolium-based colorimetric method by using the compound 3-(4,5-dimethylthiazol-2-yl)-2,5-diphenyl-tetrazolium bromide (MTT). The cytotoxic dose of 50% (CD₅₀) is the concentration of the compound which reduces the absorbance of the control sample by 50%.

### HHV 8 viral antigens detection by cytofluorimetry

The BC3 cells were treated with the compounds under test for 6-12 days. Then, the cells were washed twice with PBS (phosphate buffered saline) and fixated with frozen methanol for 4 min in refrigerator. The fixated cells were washed and treated with anti-LANA rat antibodies diluted in accordance with the manufacturer's instructions (Santa Cruz Biotech, CA, USA); the same were again washed and treated with a solution of rabbit anti-rat antibodies conjugated with FITC (fluorescein isothiocyanate). After washing, the cell fluorescence was detected in a Perkin-Helmer cytofluorimeter.

### ELISA-based P53-MDM2 binding assay

The interaction of the sulphonamide compounds of the invention with the MDM2-p53 complex was assessed according to Böttger's method (Böttger A., et al., Design of a synthetic MDM2-binding mini protein that activates the p53 response in vivo. Curr. Biol. 1997, 7(11), 860-869) with some modifications. In short, 96-well ELISA micro-plates (from Greiner Bio One, Austria) were coated for incubation overnight with 2 µg/mL p53 (human recombinant from Sigma) in PBS at 4 °C. The next day, the coating buffer was removed and the plates were incubated with Chemi-Blocker blocking buffer (from Merck-Millipore, Italy) for 2 hours at room temperature to block non-specific protein binding sites on the plate. Then, the plates were washed 4 times with PBST (10 mM phosphate buffer at pH 7.4, 150 mM NaCl, 0.05% Tween 20) before incubation with MDM2 (1.3 µg/mL) labelled with glutathione S-transferase (human GST-MDM2 from Sigma) prepared in the blocking buffer. The compounds under test were dissolved in DMSO (dimethyl sulfoxide, 5 mg/mL stock solution) and diluted in blocking buffer to obtain a final concentration of 50 µg/mL. The total incubation time of GST-MDM2 was 1 h. In order to evaluate the possible interactions of the compounds of the invention with, alternately, p53 and/or MDM2, the tests were conducted in three ways: a) pre-incubation with p53: the compounds were added to the coated micro-wells 30 min. before adding the GST-MDM2; b) pre-incubation with MDM2: the compounds were added to the GST-MDM2 solution 30 minutes before adding into the wells; c) incubation after the formation of the p53-MDM2 complex: the compounds were added to the wells 30 minutes after treatment of GST-MDM2. After incubation, the plates were washed 4 times with PBST to remove the unbound MDM2 and incubated with a specific rabbit anti-GST monoclonal antibody (Sigma) diluted 1/2000 in blocking buffer for 1 h, followed by washing and incubation with a secondary immunoglobulin antibody conjugated with 1/200 anti-rabbit HR-peroxidase (horseradish) for 1 h (goat anti-rabbit IgG-HRP, Sigma). The unbound secondary antibody was removed by washing and the plates were incubated for 30 min. with stabilized chromogen (*Stabilized Chromogen,* Invitrogen), for the secondary antibody conjugated with HRP and incubation was instantly blocked with a blocking solution (*Stop Solution,* Invitrogen). The absorbance was measured with a *Microtiter* plate reader (*Model 680,* from Bio-Rad, Hercules, CA) equipped with a 450 nm filter and the respective luminescence units (RLU) were measured. The inhibition percentage of the p53-MDM2 complex was calculated as follows:

### (RLU found in the treated compound sample - RLU of controls in DMSO) x 100

Data are expressed as µg/mL ± average SE of three independent experiments.

### Statistics

The analysis of variance was carried out by a one-way ANOVA with Fisher as *post-hoc* test. A difference was considered significant when it was p < 0.05.

### Results

### Detection of the cytotoxic activity of the sulphonamide compounds of the invention both on human primary cells and on human cell lines

The cytotoxic activity of the drugs of the present invention under test was detected on two different human cell lines (cancerous) and on a culture of (healthy) human primary cells. In said test, BC3 cells constitute a lymphoma cell line permanently infected by HHV 8, THP-1 cells constitute a macrophage cell line devoid of HHV 8 and HUVEC cells are (healthy, uninfected) primary human cells extracted from the umbilical cord.

The following Table 3 shows the results obtained with some of the preferred compounds of the present invention. In an interesting and unexpected manner, the cytotoxicity of the drugs tested was found to be different in the (healthy) primary cell lines and in cancer cells. All compounds showed a CC₅₀ of about 100 µg/mL on BC3 cells, while on THP-1 cells, CC₅₀ values were found to range from about 30 µg/mL (for the compound internally marked as ST5) to a maximum of about 125 µg/mL (for the compounds internally marked as SG3, SA4, BC6, respectively); instead, the HUVEC cells were generally more resistant to the drug cytotoxicity, showing CC₅₀ values ranging from > 125 for the compound marked as BC6, to about 250 µg/mL for the compound marked as SA4 and ST5, and about 500 µg/mL for the compound marked as SG3. So, on average, substantially in all cases the sulphonamide compounds of the present invention were unexpectedly found to be more toxic on the diseased cell lines than on healthy human primary cells.

**Table 3: Cytotoxic activity of the drugs of the invention on the primary human cell lines and on tumour cell lines**

| CC₅₀ µg/mL +/- SE | | | |
|---|---|---|---|
| Drug | BC3 cells | THP-1 cells | HUVEC cells |
| SG3 | 100.00 | 125.05 | 500.20 |
| SA4 | 100.30 | 125.40 | 250.50 |
| ST5 | 100.22 | 30.10 | 250.55 |
| BC6 | 100.20 | 125.30 | 125.60 |

| | | | |
|---|---|---|---|
| CC₅₀ : cytotoxic concentration that inhibits the cell viability by 50%. BC3: lymphoblastic cell line. THP-1: macrophage cell line. HUVEC: healthy umbilical cord human primary cells. Drugs: SG3 = sulphaguanidine; SA4 = sulphanilamide; St5 = sulfathiazole; BC6 = sulfamethoxazole. | | | |

### Antiviral activity of the sulphonamide compounds of the present invention on HHV 8 in the latent stage

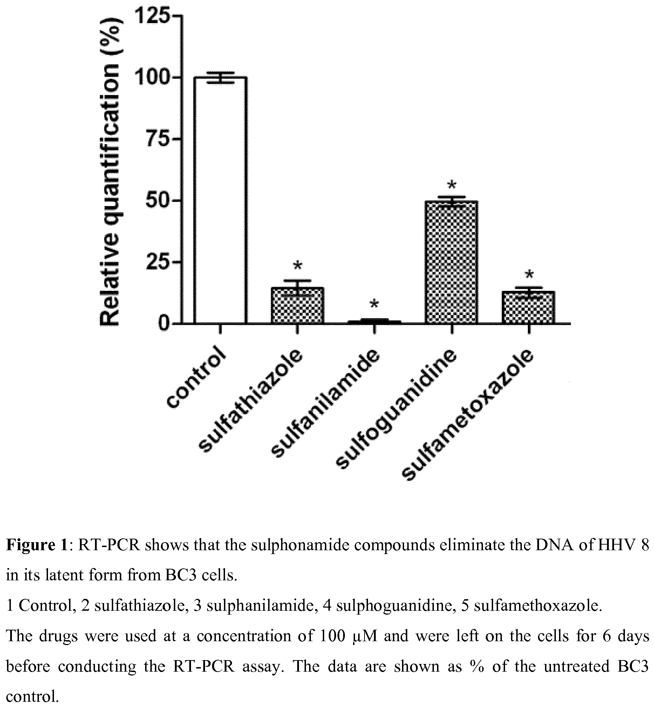

The antiviral activity of the sulphonamide compounds of the present invention was investigated using two different types of tests. In the first one, the DNA of HHV 8 in latent stage was detected by RT-PCR (Real Time- Polymerase Chain Reaction) (see Fig. 1) and in the second one, the LANA antigen was tested using a cytofluorimetric assay.

In the first test, after 6 days of cell culture in the presence of the drugs at the concentration of 100 µM, the DNA of HHV 8 was eliminated, although to a different extent, by all the compounds tested. Sulphaguanidine was found to be the least active antiviral (despite being significantly active anyhow), inhibiting viral DNA by 52.3%. In turn, sulphanilamide, sulfamethoxazole and sulfathiazole were more active, showing an antiviral activity of 92.0%, 93.0% and 88.5%, respectively.

In the second test the inhibition of the LANA antigen expression by BC3 cells after treatment for 6 days with a number of preferred compounds of the invention to the concentration of 100 µM was investigated. Also in this case, the sulphonamide compounds of the invention significantly inhibited the expression of HHV 8 antigen in latent stage compared to untreated BC3 cells; the LANA factor was reduced by 50.3% by sulphaguanidine, 79.4% by sulfathiazole, 92.0% by sulphanilamide and 93.0% by sulfamethoxazole, respectively.

### The sulphonamide compounds of the present invention affect the in vitro formation of the MDM2-p53 complex

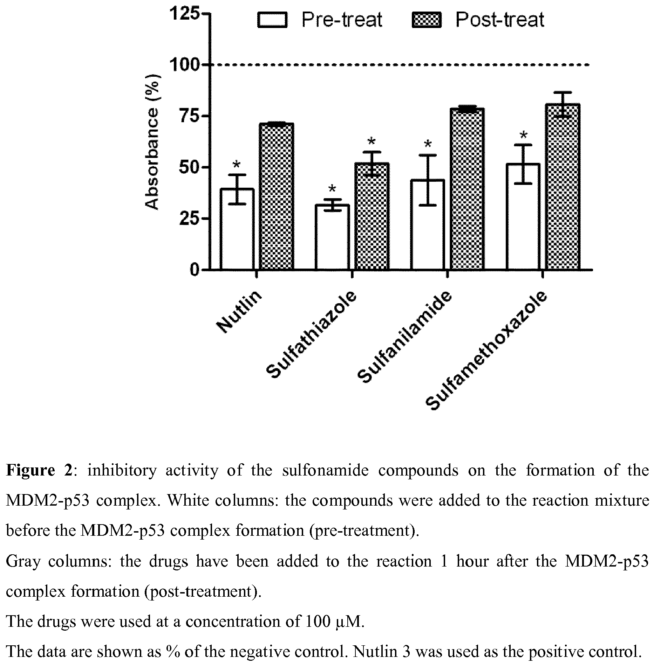

The activity of the sulphonamide compounds of the present invention on the MDM2-p53 complex was tested before and after the formation of the complex, to assess whether said compounds are capable of interfering with the formation of the complex or if they are able to break the complex when the same is already formed. Figure 2 shows that all compounds tested significantly inhibit the MDM2-p53 complex formation when they are added to the test wells before the formation of the complex. In comparison with the product nutlin 3, mentioned above and used as a positive control, sulfathiazole showed an even stronger activity (about 70% inhibition against 62% of the compound nutlin 3); also the other compounds tested showed a significant activity against the formation of the complex, with percentages of inhibition of 56% and 50%, respectively, for sulphanilamide and sulfamethoxazole. Finally, it was also found that sulfathiazole was capable of breaking down the complex already formed by about 48% (p = < 0.01), when it was added to the ELISA test wells 60 minutes after the same was formed.

The same types of tests were repeated, *mutatis mutandis* on the other sulphonamide compounds mentioned above. The preliminary results and conclusions obtained were congruent with the results described for the other compounds in the experimental section above.

### Conclusions

In the light of the foregoing, it was unexpectedly demonstrated that a number of known sulphonamide compounds used as antibacterials have a strong antiviral activity against the HHV 8 virus in its latent stage. In fact, these compounds have been shown to be able to eliminate *in vitro* the DNA of the HHV 8 virus in latent stage in permanently infected BC3 cells, thus providing the possibility to successfully and completely treat HHV 8 infection in its latent stage, while conventional anti-herpes drugs are only capable of suppressing the viral particles which reproduce actively (i.e., the virions active in the lytic stage of the virus).

In particular, the antibacterial/antibiotic sulphonamide compounds for use according to the claims have been shown to be able to inhibit the HHV 8 latency stage by interfering with the formation of the MDM2-p53 complex that is necessary for the LANA to bind to the permanently infected lymphoma cells.

Interestingly, the compounds for use according to the claims have eliminated both the DNA of the HHV 8 virus in the latent stage and the LANA antigen expression at µM concentrations and some of the drugs tested were even more active than the compound nutlin 3 used as a positive reference compound. It is also noteworthy, and wholly unexpected, as described in the previous experimental section, that the sulphonamide drugs for use according to the claims showed a selective toxicity on diseased cell lines, such as lymphoid cells as BC3 and THP-1, higher than the one shown on healthy HUVEC human primary cells. This fact highlights the possibility to advantageously use the sulphonamide compounds for use according to the claims on cell lines infected by HHV 8 at concentrations that do not exert toxic effects on normal human tissues.

As mentioned above, the sulphonamide antibacterial/antibiotic compounds for use according to the claims are substantially present and sold in most pharmacies around the world. They are therefore already well known for their pharmacokinetics, biological activity, toxicity and dosage and also their potential complications are known and considered. Therefore, their use for the treatment of proliferating diseases caused by/attributable to the HHV 8 virus in latent stage is potentially fast and easy.

### Industrial Applicability

The tests carried out on the sulphonamide compounds for use according to the claims have demonstrated that they have the ability to interfere with the expression of the factors that regulate the latency state of the HHV 8 virus and thus cause the elimination thereof from cells *in vitro.* These tests were confirmed on primary cells and cell lines and a drug treatment has been developed that can completely eradicate the HHV 8 virus from infected cells.

In conclusion, said sulphonamide compounds/drugs, already used as antibacterial drugs, are able to eliminate the HHV 8 virus in its latent stage from permanently infected BC3 cells, thus providing the ability to permanently cure the proliferating diseases due to/attributable to the HHV 8 virus in the latent stage, in particular diseases such as Kaposi's sarcoma, primary effusion lymphoma (PEL) and multicentric Castleman disease (MCD). These compounds can be used immediately, even without additional clinical trials, both as a complementary therapeutic treatment of conventional anti-HHV 8 infections and also as a single therapy to completely cure the latency form of the HHV 8 virus or, possibly, even for the preparation, or as adjuvants for use, of a specific anti-HHV 8 vaccine.

## Claims

1. A sulphonamide compound with antibacterial/antibiotic activity, selected from the group consisting of sulphanilamide, sulphaguanidine, sulfathiazole, sulfamethoxazole and/or mixtures thereof, for use in the treatment of KSHV/HHV 8 virus in its latent stage and/or for use in the treatment of diseases caused by said virus or attributable thereto, wherein said diseases caused by said virus or attributable thereto are selected from Kaposi's sarcoma, pleural effusion lymphoma (PEL), and Castleman's disease.

2. A pharmaceutical composition comprising at least one of the sulphonamide derivatives of claim 1 as an active ingredient, for use in the treatment of KSHV/HHV 8 virus in its latent stage and/or for use in the treatment of diseases caused by said virus or attributable thereto, wherein said diseases caused by said virus or attributable thereto are selected from Kaposi's sarcoma, pleural effusion lymphoma (PEL), and Castleman's disease.

3. A pharmaceutical composition for use according to claim 2, wherein said at least one active ingredient is present in an effective amount of between 100 and 1,000 mg.

4. A pharmaceutical composition for use according to any one of claims 2 or 3, wherein the daily dose of the active ingredient(s) is in the range from 200 to 2,000 mg of active ingredient(s) *pro die,* divided into 1-3 administrations.

## Patentansprüche

1. Sulfonamidverbindung mit antibakterieller/antibiotischer Wirkung, ausgewählt aus der Gruppe, bestehend aus Sulfanilamid, Sulfaguanidin Sulfathiazol, Sulfamethoxazol und/oder Gemischen davon, zur Verwendung bei der Behandlung des KSHV/HHV 8-Virus in seinem latenten Stadium und/oder zur Verwendung bei der Behandlung von durch das Virus verursachten oder ihm zurechenbaren Krankheiten, wobei die durch das Virus verursachten oder ihm zurechenbaren Krankheiten ausgewählt sind aus Kaposi-Sarkom, Pleuraerguss-Lymphom (PEL) und Castleman-Krankheit.

2. Pharmazeutische Zusammensetzung, umfassend mindestens eines der Sulfonamidderivate nach Anspruch 1 als Wirkstoff zur Verwendung bei der Behandlung des KSHV/HHV 8-Virus in seinem latenten Stadium und/oder zur Verwendung bei der Behandlung von durch das Virus verursachten oder ihm zurechenbaren Krankheiten, wobei die durch das Virus verursachten oder ihm zurechenbaren Krankheiten ausgewählt sind aus Kaposi-Sarkom, Pleuraerguss-Lymphom (PEL) und Castleman-Krankheit.

3. Pharmazeutische Zusammensetzung zur Verwendung nach Anspruch 2, wobei der mindestens eine Wirkstoff in einer wirksamen Menge zwischen 100 und 1000 mg vorhanden ist.

4. Pharmazeutische Zusammensetzung zur Verwendung nach einem der Ansprüche 2 oder 3, wobei die Tagesdosis des/der Wirkstoff(e) im Bereich von 200 bis 2000 mg Wirkstoff(e) pro Tag, verteilt in 1-3 Verabreichungen, liegt.

## Revendications

1. Composé sulfonamide à activité antibactérienne/antibiotique, choisi dans le groupe consistant en le sulfanilamide, la sulfaguanidine, le sulfathiazole, le sulfaméthoxazole et/ou leurs mélanges, à utiliser dans le traitement du virus KSHV/HHV 8 à son stade latent et/ou à utiliser dans le traitement de maladies provoquées par ledit virus ou qui lui sont attribuables, dans lequel lesdites maladies provoquées par ledit virus ou qui lui sont attribuables sont choisies parmi le sarcome de Kaposi, le lymphome à épanchement pleural (PEL), et la maladie de Castleman.

2. Composition pharmaceutique comprenant au moins l'un des dérivés sulfonamide de la revendication 1 comme ingrédient actif, à utiliser dans le traitement du virus KSHV/HHV 8 à son stade latent et/ou à utiliser dans le traitement de maladies provoquées par ledit virus ou qui lui sont attribuables, dans laquelle lesdites maladies provoquées par ledit virus ou qui lui sont attribuables sont choisies parmi le sarcome de Kaposi, le lymphome à épanchement pleural (PEL), et la maladie de Castleman.

3. Composition pharmaceutique à utiliser selon la revendication 2, dans laquelle ledit au moins un ingrédient actif est présent dans une quantité efficace entre 100 et 1 000 mg.

4. Composition pharmaceutique à utiliser selon l'une quelconque des revendications 2 ou 3, dans laquelle la dose quotidienne du ou des ingrédient(s) actif(s) est dans la plage de 200 à 2 000 mg d'ingrédient (s) actif (s) *par jour,* divisée en 1 à 3 administrations.
